# EUROPEAN PATENT APPLICATION

(11) **EP 3 001 340 A1**
(43) Date of publication of application: **30.03.2016**
(21) Application number: 15186127.5
(22) Date of filing: 21.09.2015
(51) Int. Cl.: G06F 19/00

(54) **MEDICAL IMAGING VIEWER CACHING TECHNIQUES**

(30) Priority: 29.09.2014 US 201414500027
(71) Applicant: Vital Images, Inc., Minnetonka, MN 55343 (US)
(72) Inventor: NYE, Jason, Minnetonka, MN 55343 (US)
(74) Representative: Collins, John David

(57) **Abstract**

Techniques for client-side caching of a medical image and server-side coordination of the client-side caching techniques are disclosed herein. User interaction in a client medical image display interface is coordinated with server conversion and processing operations. In one example, a server provides a unique key and display parameters to coordinate and specify client display operations. The unique key can be used to obtain a copy of the image from a local client-side cache, and the display parameters can be used to specify client-side transformation and rendering techniques to implement a requested image view. As a result of the client-side caching operations, resource utilization (including computational processing and network bandwidth) can be minimized, while user interactivity can be maximized.

## Description

### TECHNICAL FIELD

Embodiments pertain to techniques and systems for displaying images obtained from medical imaging procedures. Further embodiments relate to processing and caching techniques in medical imaging viewer software applications.

### BACKGROUND

Existing medical imaging viewers provide functionality for the display of medical images obtained from medical imaging procedures, such as the display of image data produced from radiology imaging scans (e.g., x-ray, MRI, CT, ultrasound imaging procedures), pathology imaging, and like diagnostic imaging procedures. Many medical imaging viewers currently in use enable the display and rendering of medical imaging data within web browsers. Medical imaging viewers that are developed for web browsers include "zero-footprint" viewers, which enable the display of subject images in a browser at a remote location without the use of plug-ins or proprietary client software. The functionality provided by many zero-footprint viewers, however, is often limited based on the browser-server communication model that makes repeated requests of the server for browser-compatible images as the user interacts with the imaging viewer.

### BRIEF DESCRIPTION OF THE DRAWINGS

FIG. 1 illustrates a block diagram of a system configuration operable for processing and distributing medical imaging data among a medical imaging system, image processing server, and a client browser system according to an example described herein.
FIG. 2 illustrates a block diagram of a system configuration operable for processing and caching medical imaging data between an image processing server and a client browser system according to an example described herein.
FIG. 3 illustrates an association between a set of display parameters and a display area for display of a portion of a medical image used in connection with caching medical imaging data according to an example described herein.
FIG. 4A illustrates a screen shot of a graphical user interface of an image viewer in a carousel image display mode operable in a client browser system according to an example described herein.
FIG. 4B illustrates a screen shot of a graphical user interface of an image viewer in a multi-cine playback mode operable in a client browser system according to an example described herein.
FIG. 5 illustrates an operational sequence of client and server techniques for performing caching with a medical imaging viewer according to an example described herein.
FIG. 6A illustrates a flowchart of a technique for performing caching processing operations in a medical imaging viewer according to an example described herein.
FIG. 6B illustrates a flowchart of a technique for performing processing operations at a server supporting caching for a medical imaging viewer according to an example described herein.
FIG. 7 illustrates a system configuration diagram of processing and viewer systems arranged to perform caching of converted medical images according to an example described herein.
FIG. 8 illustrates an example of a machine configured to perform computing operations according to an example described herein.

### DETAILED DESCRIPTION

The following description and the drawings sufficiently illustrate specific embodiments to enable those skilled in the art to practice them. Other embodiments may incorporate structural, logical, electrical, process, and other changes. Portions and features of some embodiments may be included in, or substituted for, those of other embodiments.

The present disclosure illustrates various techniques and configurations that enable the use of client-side caching of medical images in a web browser-based medical imaging viewer. These include the use of caching techniques within objects and data stores of a web browser that provides an advanced rendering of medical images based on JavaScript, HTML5, and other dynamic browser display functionality. This cache may be populated and cleared dynamically based on the needs of the client, and managed separately from the default browser caching mechanism. The present disclosure further illustrates techniques and configurations for an application web server that coordinates operations of the image display operations, enabling the server to track the characteristics and locations of displays on the client, and control whether the client caches or persists a particular image.

Many existing web browser-based medical imaging viewers operate with the use of server-rendered images. For example, systems may implement an image-by-image rendering scheme, such that if a client only requests a middle image in a large stack of images, only that middle image is rendered at the server and served to a viewing client browser. In many scenarios, if any change or manipulation to the viewing parameters is made in the image viewer (e.g., a change in brightness/contrast viewing window, scale, panning, or the like) at the client, the server will need to re-render a copy of the image to reflect the appropriate changes and deliver a new copy of the image to the client viewer. In some scenarios, the desired image will need to be re-transmitted to the client, even if the desired image was previously displayed at the client.

Medical imaging viewers may also provide a "playback" mode, often referred to as a "CINE" mode, which plays back, loops, or cycles an image sequence according to a particular frame rate. In particular, two sets of medical imaging series may be placed next to each other in a playback mode, side-by-side in a graphical user interface, to allow a medical professional to directly compare two or more imaging series that are synchronized according to some common feature. For example, a series of images of common anatomy that is captured at two different times, with or without contrast, and the like may be played back in a multi-cine mode.

In such a multi-cine mode, if any change or manipulation is made to the parameters of the browser image viewer, such as the resizing of a first set of images, the second set of images that are being displayed on the client will become "out-of-sync" with the changes made to the first set of images. In many cases, this may lead to an incorrect display in the multi-cine mode, or the loss of synchronization or other playback functionality between the compared imaging series. This along with other image manipulations performed in the image viewer may necessitate new sets of images to be downloaded from the server. This may lead to additional download activity from the server, and additional processing at the client and the server.

The techniques described herein provide for the establishment of a client-side (e.g., browser-based) cache that is used to persist a copy of images downloaded from the server. This client-accessible cache is accompanied by techniques to maintain a correspondence of the image and image display characteristics to data maintained at the server. With the use of the presently described cache and a tracking mechanism, the state of image viewing operations performed at the client can be synchronized by the server, resulting in enhanced processing characteristics at both client and server locations. In this fashion, the client can act as a passive participant, allowing the server to determine when client side image updates are necessary.

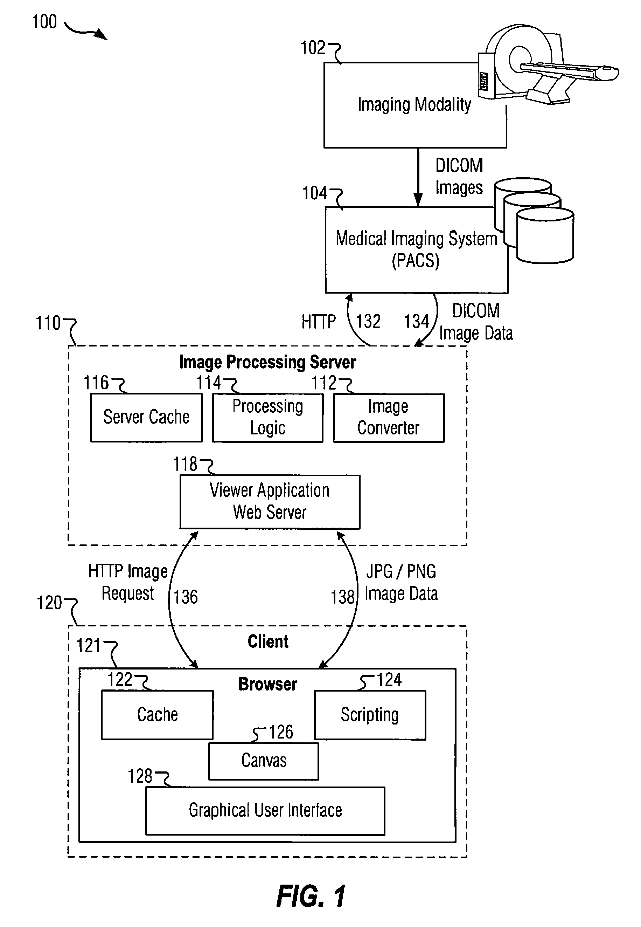

For purposes of illustration, the medical imaging environment configuration 100 depicted in FIG. 1 includes an imaging modality 102, a medical imaging system 104 (e.g., a Picture Archiving Communication System (PACS)), an image processing server 110, and a client computer 120. It will be understood that the following environment description is provided as an example, and individual features of the following medical imaging system and imaging processing server may be integrated or separated among various machines, systems, and devices. Further, many of the client-server models depicted in the following examples may be simulated or replicated by like functions within device subsystems.

As shown, medical imaging data (e.g., a series of images) is produced from a medical imaging procedure (e.g., a radiological scan from a computed tomography (CT) scanner) conducted at the imaging modality 102. The medical imaging data is provided for storage within the medical imaging system 104 (e.g., a Picture Archiving and Communication System (PACS)). The medical imaging system 104 may integrate with electronic medical record (EMR) systems, electronic health records (EHR) systems, and other electronic medical information systems which store, maintain, and access medical imaging data obtained from and associated with the medical imaging procedure. The format of the electronic data produced by the imaging modality may be in a proprietary or industry standard format, such as in a Digital Imaging and Communications in Medicine (DICOM) format). Typically this imaging modality format is not natively readable by web browsers.

The image processing server 110 operates to receive and process image data, e.g., DICOM image data 134, obtained from the medical imaging system 104. As discussed herein, the DICOM image data 134 may be processed and converted by the image processing server 110 for use in a client viewer. The image processing server 110 includes an image converter 112, processing logic 114, a server cache 116, and a viewer application web server 118. The viewer application web server 118 operates to receive HTTP requests from clients (e.g., the client computer 120) and respond with data compatible for rendering at the client computer 120. For example, the viewer application web server 118 may operate to serve HTML data, JPEG/PNG data, and other data compatible for display in a web browser universal image viewer (e.g., the browser 121 operating on the client computer 120).

On the image processing server 110, the image converter 112 operates to receive unconverted image data (e.g., the DICOM image data 134) and convert the image data into another format or representation (e.g., into JPEG/PNG image data 138). The processing logic 114 operates to respond to requests for specific image locations from the client computer 120, and facilitate the conversion of the particular unconverted image data with the image converter 112. In some examples, a request 132, transmitted over HTTP (e.g., a request in the format of a Medical Imaging Network Transport (MINT) request) may be provided to the medical imaging system 104 to request and obtain copies of the images in real-time. In other examples, a server cache 116 or other data store operates to obtain and persist copies of the images, before or after conversion. For example, image converter 112 may operate to convert and generate copies of a plurality of images from a particular study, a particular patient, a particular procedure, a particular medical facility, or the like for storage in the server cache 116, allowing the image processing server 110 to pre-fetch and convert sets and series of the images. In other examples, the server cache 116 may store and serve only cache images that are specifically converted by the image converter 112 in response to a user command or request from the client computer 120.

The client computer 120 operates a client application used to display images generated by the image processing server 110. In one example, the client application operates within a HTML browser 121, which provides a zero footprint, zero download deployment for the user. For example, the client application may operate with interactive functionality without the need for plugins (e.g., Flash, Java applets, Silverlight, etc.) within the HTML browser 121. The client application therefore may operate within a standard featured browser, utilizing dynamic webpage viewer technologies including JavaScript, HTML5, and other browser native interaction specifications, to enable a dynamic manipulation, interaction, and regeneration of displayed content within the HTML browser 121. Accordingly, the client application may be designated for a browser- and platform-agnostic operation, with operations of the client application adaptable to a variety of operating system and browser types that provide a full-featured browser.

The client application operating with the browser 121 may interact with a variety of built-in or custom functions. For example, the browser 121 may include or may be integrated in operation with a client-side cache 122, a scripting function 124, a canvas 126, and a graphical user interface 128. As a result of interactions received in the graphical user interface, the user may perform various scripting actions. This may result in an image request, such as an HTTP image request 136, being provided to a viewer application web server 118. The viewer application web server 118 may respond with the converted image data (such as JPEG or PNG image data 138). The scripting function 124 may operate to determine the interaction with the canvas 126 and request image data from the viewer application web server 118 via the HTTP image request 136 (and request the image data from the client-side cache 122 if available). The canvas 126 then operates to redraw the appropriate output of the image data and provide interactivity via the graphical user interface 128.

The client-side cache 122 operates to provide a client-side persistence of relatively-infrequently changing (or constant) images. The client-side cache 122 is designed for repeated access by the scripting 124, to allow a display of cached images in the canvas 126. In some examples, the client-side cache 122 may be implemented as a binary object (e.g., in JavaScript) having a table of key-value pairs, with the key being an image key and the value being a representation of the image uniquely described by the key. As further described below, the client-side cache 122 may include a design to manage the storage of image data. For example, the client-side cache 122 may be configured with a maximum size (in bytes) that it will store before it starts evicting cached images.

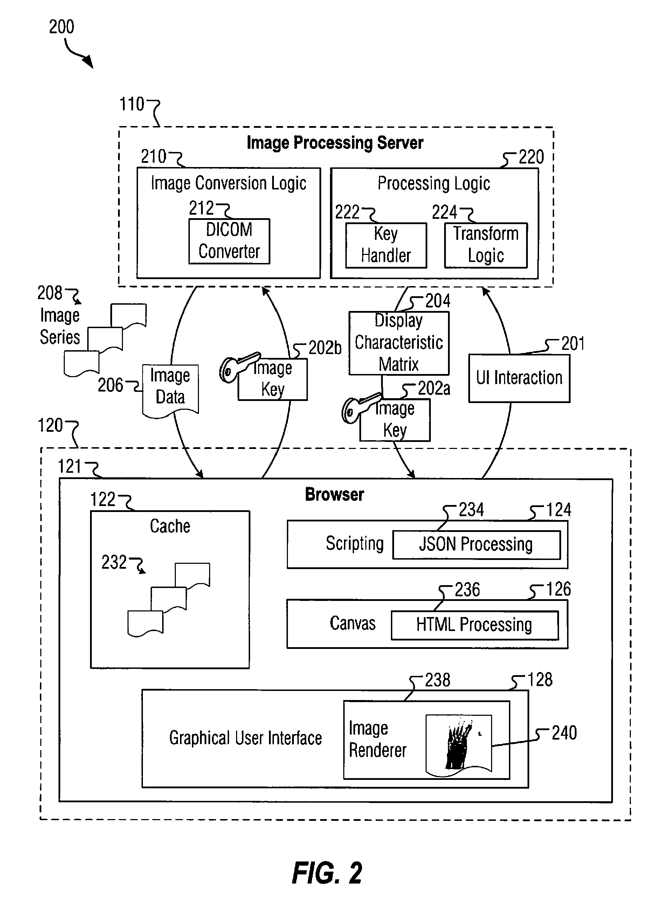

FIG. 2 provides further illustration of a client/server configuration 200 providing processing functionality between the image processing server 110 and the browser 121 of the client computer 120. As shown, the image processing server 110 provides a unique identifier of a particular image in an image key 202a and provides display parameters of the particular image in a display characteristic matrix 204, in response to client user interface interaction 201 providing requests and data to the server. The image processing server 110 further provides image data 206 for an image in an image series 208, which may be provided in response to a request for the images provided with the image key 202b (the image key 202b matching the image key 202a previously provided to the client computer 120). The image key 202a, 202b is used to identify a particular image for retrieval and display, and the display characteristic matrix 204 is used to identify display parameters on the client of that particular image.

At the image processing server 110, image conversion logic 210 operates to convert image data for display at the client computer 120. For example, the image conversion logic 210 may utilize a DICOM converter 212 to convert DICOM image data into a JPEG/PNG/other web browser-compatible format. Also at the image processing server 110, processing logic 220 provides specific selection and processing for the requested image. A key handler 222 operates to receive and process requests for images and image data via an image key. For example, the image key may be provided within a URL (e.g., as part of the URL) request to the image processing server 110. Transform logic 224 operates to determine and track the appropriate portion of the image and display characteristics of the image that is desired for output on the client computer 120, indicated by the display characteristic matrix 204. The converted image may be a high-resolution JPEG/PNG format that allows a single high-quality image to be produced at the server and transmitted to the client-side cache, and then scaled, positioned, and manipulated on the client after being stored at the client-side cache.

At the client computer 120, the browser 121 operates with the use of the scripting 124 and the canvas 126 to provide dynamic output in the graphical user interface 128. For example, the scripting 124 may initiate events and requests to the server (e.g., "image update" requests) in response to detected conditions in the browser 121 such as navigation or selection of another image or portion of the image. The scripting 124 may include a JSON (JavaScript Object Notation) processing element 234 that processes JavaScript elements performing requests and processing of data within the browser 121. The canvas 126 may include a HTML processing element 236 which provides a dynamic display and manipulation for a display of the browser 121, for output in the graphical user interface 128. The graphical user interface 128 may include an image renderer component 238 for display of the browser image data (e.g., JPEG/PNG data), such as a specific view of a medical image 240.

The scripting 124 may operate to retrieve image data maintained with the client-side cache 122. For example, if the desired image (e.g., the medical image 240) to be displayed is stored in the client-side cache 122, the scripting 124 operating at the client may retrieve the image data from the client-side cache 122 using the image key 202a. In this caching scenario, the browser will first indicate the user interface interaction 201 to the image processing server 110, and the image processing server 110 will provide an identifier (the image key 202a) to access the image stored locally in the cache 122. From this response, the scripting 124 can retrieve the information for the medical image 240 from the cache 122, and perform suitable transformation and selection of the image on the client according to the parameters specified in the display characteristic matrix 204. The medical image 240 may be stored individually or part of a series of cached images 232, allowing one or multiple images of the cached images 232 to be retrieved locally at the client computer 120 without re-transfer of the image data from the image processing server 110.

In determining the particular characteristics of the client-side display, the display characteristic matrix 204 is provided according to operations of the transform logic 224 at the server. The information in the display characteristic matrix 204 is communicated from the server with use of the transform logic 224, operating to specify display parameters that ensure that the server is fully aware and in control of client display actions. From this, the server operates to coordinate and manage interactions between different series and types of images. Accordingly, both server-side and client-side bandwidth and processing can be coordinated.

With use of the client-side cache 122, user interactions that affect the scale or position of the image need not result in new image transfers from the image processing server 110, once the images have been cached on the client in the client-side cache 122. In some examples, only the display orientation information (included in the display characteristic matrix 204) and the key identifying the image (included in the image key 202a) are transferred across the network during interactive zooming, panning, and other manipulations occurring in the graphical user interface 128 upon a cached image. When switching to another image, the image renderer component 238 can simply update its rendering of the particular image (e.g., displayed medical image 240) to another entry of the cached images 232 stored in the client-side cache 122. Thus, cached images may serve as "base images" that can be re-used with different display parameters applied to the images.

This caching mechanism provides benefits, for example, when the user at the graphical user interface 128 is viewing a large number of relatively small images or a small number of large images. For example, when the user is viewing a large number of relatively small images using the client-side caching, the user can navigate and scan through these images repeatedly without causing the images to be re-downloaded from the server. This allows a reduction in bandwidth and remote processing, even when the user interaction primarily involves zooming or panning to different areas of the images. Likewise, when the user is viewing a small number of large images, the user typically stays on a specific image longer. User interaction for zooming or panning the cached image will not require a re-download from the server, making the interactivity and the client side more responsive. In these and other scenarios, the client-side cache 122 can be designed to hold a significant number of images, greatly reducing the need to re-download images from the server as manipulations occur in the graphical user interface 128.

The cache 122 may be configured to store data as a table of key-value pairs separately from any browser cache, the key being the image key (e.g., the image key 202a) to identify a particular image in the cache, and the value being a representation of the image uniquely described by the key (e.g., JPEG/PNG data or a link to this data). The cache may be configured with a maximum size (in bytes) that it will store before it starts evicting images. For example, a least-recently-used algorithm may be used to remove copies of older data. Such a system enables storing an image recently retrieved from the server may cause one or more currently cached images to be evicted, requiring them to be re-downloaded from the server if accessed again.

Other algorithms based on characteristics of the image or image series also may be used to manage the cache storage. In other examples, the cache storage may be managed based on a usage model or an adaptive model, so that if space is required client-side, images will be evicted as needed. In any event, even if the cache is purged, the images may be re-downloaded and introduced back into the cache if required at a later time. (While opaque to the client, the server knows how to accurately re-generate the image data for the particular key when necessary.)

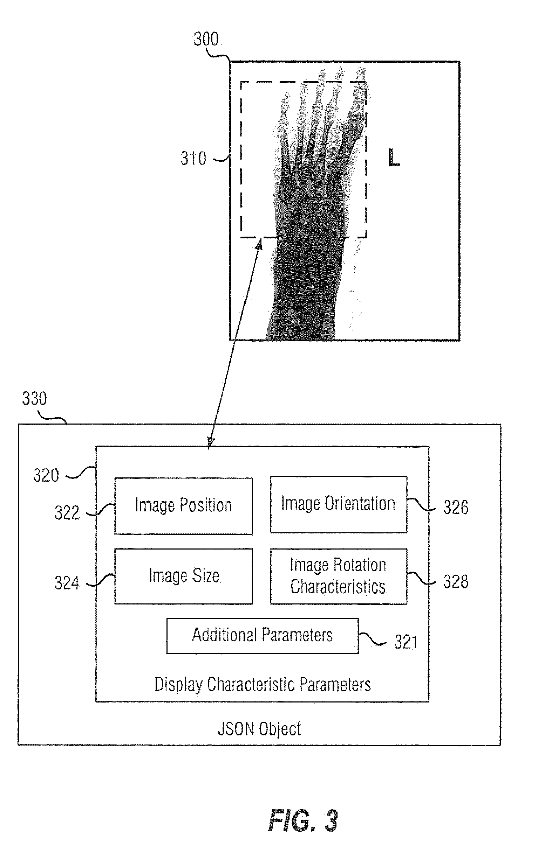

FIG. 3 provides a further illustration of the display characteristics used for accessing and retrieving imaging data according to the system operation examples described herein. As shown, an output of a graphical user interface includes the output of a display portion 310 for a radiological image 300. For example, the display portion 310 may correspond to a visible output in a client graphical user interface (e.g., graphical user interface 128).

The display portion 310 of the graphical user interface is coordinated to value of display parameters 320 maintained within a JSON object 330. This JSON object 330 is maintained by the client side scripting elements (e.g., JSON processing element 234), and implements the server specification of the characteristics of the display portion 310. For example, the values indicated in the display parameters 320 may control and track: an image position 322 (e.g., an indication of the visible portion of the image within a larger image); an image size 324 (e.g., the image size output in pixels, such as 1024x1024, displayable in the display portion 310 of the graphical user interface); an image orientation 326 (e.g., an absolute orientation of the image 300 in the display portion 310 of the graphical user interface) and image rotation characteristics 328 (e.g., rotation characteristics of the image 300 in the display portion 310 of the graphical user interface). Other additional parameters 321 as specified by the server (or requested by the client) may also be included in connection with the display parameters 320.

In the client-side graphical user interface, the display portion 310 of the image 300 is rendered with the orientation and presentation characteristics as described by the server and designated within the display parameters 320. Based on the rendering parameters in the display parameters 320, the client is thus informed on how to render the image. Accordingly, the client renders the image 300 using the binary image data combined with the display parameters for the image render request.

In further examples, the display parameters 320 may be accompanied by other data fields relevant to the display in the graphical user interface. These may include data for brightness and contrast settings, geometric information, navigation characteristics, display preferences, and the like. For example, these settings may be used to convey a server indication for window/level values, or modifications to the window/level values made at the client by the user in the graphical user interface. A variety of other transformations and changes to the transmitted image may be implemented at the client through the use of the display parameters 320, the JSON object 330, or other dynamic display elements.

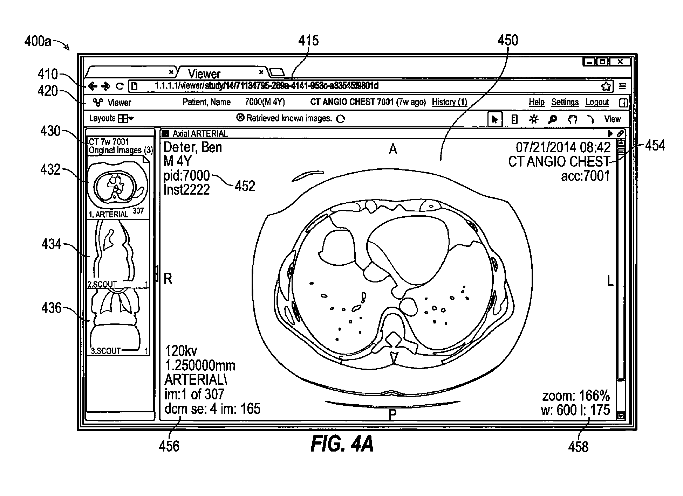

FIG. 4A provides an illustration of a graphical user interface 400a for a medical imaging viewer 420, such as a medical imaging viewer operating with the example cache retrieval techniques described herein to access and display an image from a client-side cache. As shown, the medical imaging viewer 420 operates in a "carousel" mode within in a browser window 410 of a web browser, and provides a full-featured user interface for the selection, navigation, display, and display modification for one or more images of one or more medical imaging series (e.g., medical imaging series 432, 434, 436). In an example, the user interface 400a may operate directly within the built-in functionality of the web browser, through the use of web-based technologies (e.g., HTML5, JavaScript).

As an illustrative example, the medical imaging viewer 420 may include an image listing 430 for images of a medical diagnostic imaging procedure (e.g., a CT radiology scan or scans of a particular patient). The medical imaging viewer 420 may display a plurality of thumbnails for the medical imaging series 432, 434, 436 of this medical diagnostic imaging procedure. The display of a particular imaging series (e.g., an Axial Arterial CT scan series) from the imaging procedure may be provided by the output of a single image 450 of a plurality of images from the medical imaging series 432. Although one image (image I of 307, shown in image 450) is depicted as the output of the viewer 420, it will be understood that the graphical user interface 400a can allow the display of other images in the selected image series, and can allow the display of multiple images in other display modes.

The graphical user interface may also provide additional output characteristics regarding the medical image for display, including patient identification information 452, image information 454, image series information 456, and display characteristics 458. The display characteristics 458 may include an indication of display values such as the zoom (relative to the original image) and window/level contrast values of the output image display. The medical imaging viewer 420 may also include functionality to allow navigation among images of the imaging series (e.g., outputting different images among the 307 images of the medical imaging series 432) and modification of display characteristics (e.g., changing the zoom and window/level values). This navigation and display characteristic modification may be implemented in the web browser through the use of the client-side browser dynamic display technologies described herein. In some examples, however, navigation and display characteristic modification may be accomplished through the use of additional software elements integrating into the browser, such as plug-ins, browser extensions, or like executables.

As shown, the medical imaging viewer 420 within the browser window 410 is accessed through the use of a URL 415. This URL may be a unique internet (or internal network) address of a website that serves images from a remote server or system, and this website may enable access to an imaging viewer portal, a particular set of imaging studies, a particular set of images, or a particular image. The output of the medical imaging viewer 420 provided at the unique web location may be generated in connection with the presently described client-side caching techniques. For example, the image 450 displayed from the medical imaging series 432 may be stored at the client in a client-side cache after its retrieval from the remote server. Subsequent commands to display the image 450 in the browser window 410 may involve retrieval from the client-side cache (and related server-side coordination of the display characteristics for the output of the image 450 in the browser window).

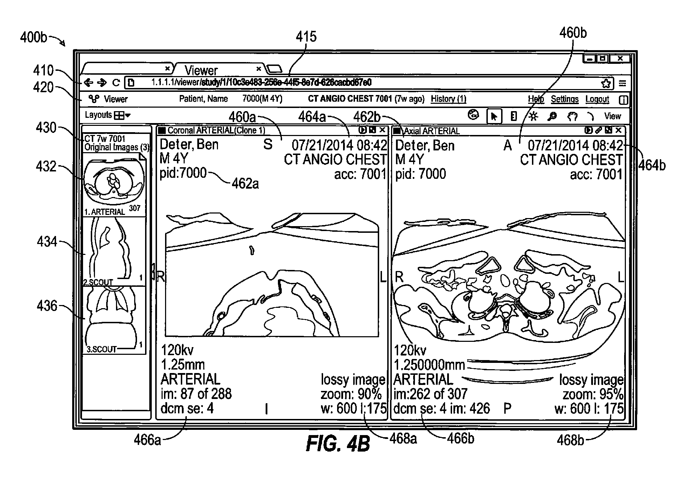

FIG. 4B provides an illustration of a graphical user interface 400b providing the retrieval of images in a multi-cine mode according to an example, such as a medical imaging viewer operating with the example cache retrieval techniques described herein to access and display multiple images from a client-side cache. As shown, FIG. 4B depicts the medical imaging viewer 420 including similar user interface functionality to that described for (and depicted in) FIG. 4A. However, in the multi-cine mode of FIG. 4B, an output of two sets of images 460a, 460b, can be provided in a side-by-side manner. Accordingly, the example output characteristics of the medical image, including the patient identification information 462a, 462b, image information 464b, 464b, image series information 466a, 466b, and display characteristics 468a, 468b, are provided respectively on the first displayed image (image 460a) and the second displayed image (image 460b).

In the multi-cine mode of FIG. 4B, an autonomous play mode may allow the playback of the images (the first displayed image, image 460a, and the second displayed image, image 460b) in sequence, to allow the playback of two series of images at the same time in synchrony. The presently described client-side caching techniques may also be used to playback a plurality of images in the multi-image mode, especially with use in a multi-cine playback mode where a series of images are repeatedly cycled and displayed.

Without use of a client-side caching, to facilitate a multi-cine mode, the client would need to repeatedly request and download each image (resulting in significant bandwidth and processor demands). Existing client-side buffering of images in the browser may enable an "autonomous play mode", where once the images have been buffered on the client, the cine loops play locally with no server interaction. Buffering in this manner has drawbacks, in that there is no mechanism to synchronize the two disparate series being played. (For instance, when using a multi-planar reformat in an autonomous play mode, reference lines cannot be correctly drawn on the views to indicate where one plane is being displayed relative to another. Because the viewports are unaware of each other's state in the autonomous play mode, various reference lines cannot be correctly shown on the images.) Thus, without use of server-side coordination, the display characteristics of the images cannot be changed or managed at the client-side. Even with client-side buffering of images in an autonomous play mode, existing systems are unable to playback two image series in full synchrony.

With the presently described caching techniques, the bandwidth and processor load can be dramatically reduced, so it is feasible for the server to remain in control of the display characteristics during the multi-cine loop. Thus, the display characteristics can be managed and indicated by the server with simple image requests between the client and the server, and with retrieval of underlying image from the client-side cache. With use of the presently described caching techniques, a multi-cine mode can be configured to enable playback of the appropriate display characteristics in full synchrony, and to include reference lines and other markup that require synchronization of the viewing portal.

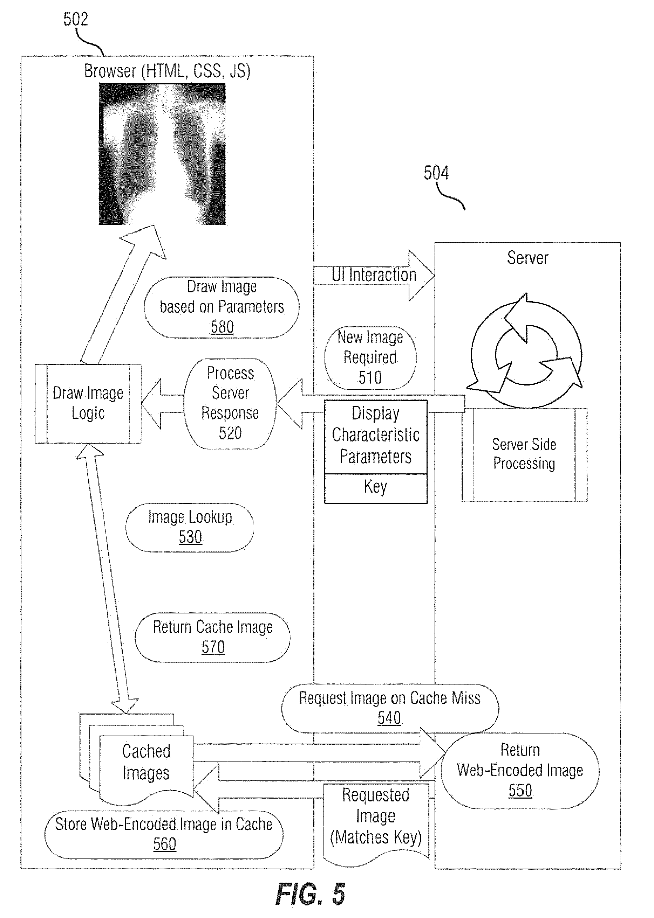

FIG. 5 provides an illustrated sequence 500 of interaction and processing operations occurring at a client browser 502 and a server 504, in connection with a client-side caching technique according to one example. As shown, the illustrated sequence 500 occurs directly between the client browser 502 and the server 504. However, it will be understood that other intermediaries and processing components may operate to facilitate the following operations.

In an example operational sequence, various image display operations occur at the browser 502 in communication with the server 504, which may include operations such as client-side interaction with an image box in a graphical user interface, for example. This request may include the communication of image display information for the image display operations occurring in the client viewer. The server side processing application at the server 504 then determines whether an image update for the image box is required to fulfill the client interaction event. For example, an image update to transfer additional data to the client can be performed as a result of user interaction for selecting a particular display of a particular image, such as provided from an image navigation, an image box resize, or some other event at the client-side graphical user interface (e.g., in the browser 502) that changes how an image (or which image) should be displayed.

In response to the interaction, the server-side processing application provides a response to the client in a new image update event 510 to the browser 502. This new image update event includes the key describing the image designated to be displayed on screen (e.g., an identifier correlated to, and referencing, the rarely-changing or constant, cacheable information), rendering parameters (e.g., how the image is to be displayed in the image box with the display characteristic matrix), and in some cases, caching instructions (e.g., an indicator to inform the client whether the image should or should not be cached). If the client browser supports parsing in-line image data (data URIs), the server-side application can inline intermediate or full versions of the image data in this new image update event to avoid an additional round trip if the server deems this necessary. For example, the server may track whether the image has been recently served to the client.

The image key is used at the client to consistently refer to an identical, converted medical image, should it need to be regenerated or re-accessed at the server. In some examples, the converted image that the image key refers to is of a fixed size and resolution. For example, the converted image may match the original DICOM image characteristics (e.g., image size dimensions), and any transformations for display output sizes may occur at the client. In other examples, some types of transformations of the image occur at the server, and the image key is used to identify a specific transformed version of the underlying image.

The display (e.g., rendering) parameters provided by the server in the new image update event 510 may include orientation information that is used to describe how the image is to be displayed in the client-side image box. This rendering information may be based on server-side geometry calculations to describe the exact position, scaling, rotation, flipping, or the like to be applied to the image when displaying the image at the client on-screen. (For example, such orientation information can inform the client how to modify the image in the browser, so that the image properly fits the display and fulfills the user's interactions (such as for zoom and panning)).

As shown in sequence operation 520, data from the new image update event is dispatched to the draw image handler located at the browser 502 for processing (including for processing of server display characteristic rendering parameters). In sequence operation 530, the draw image handler looks in the client-side cache for the image to be displayed. This cache lookup is based on the unique key exchanged between the client and server.

In sequence operations 540, 550, and 560, additional server interaction activities are conducted if a cache miss occurs. (If a cache miss occurs this means the image associated with the key is not already located in the client-side cache.) In sequence operation 540, the client retrieves the image from the server through an HTTP request, such as with use of a key provided within the server's image request URL. In sequence operation 550, in response to the image request URL, the server retrieves or re-generates the image, producing and serving an image encoded in a web-friendly format. Thus, the cache key is used to accurately reproduce the web-based format pixel data for the image.

In sequence operation 560, the returned image is stored in the client-side cache. Storing the image in the client-side cache may involve evicting one or more images from the cache to make room for the new image, as necessary. As has been explained above, subsequent fulfilled look-ups in the cache for the image, using the same image key, can avoid a future request to the server.

In sequence operation 570, the image to be used in the image update event is returned from the cache to the draw handler. (In other examples, if the cache miss occurs and an image is obtained from the server, this image is directly provided to the draw handler and the image is later stored in the cache). In sequence operation 580, the draw handler clears the image box (e.g., an HTML5 canvas), sets the canvas's transform using the orientation information supplied in the new image event, and then draws the image according to the display parameters applied to the base image by the client. This ensures the image is drawn with the orientation and parameters as described by the server.

By obtaining the image display parameters from the server, the server is in control of determining the size and position of the image to send and facilitate display. For example, the image may be available at different resolutions, such that the returned image which is selected by the server is the appropriate resolution for the display output. Additionally, the server can ensure that the orientation information sent with the new image event accurately places the image in the client side image box according to known display rules or properties.

In addition, with use of these techniques, most user interactions do not result in new images being transmitted from the server once the images have been transferred once and cached on the client. This means only the display rendering parameters and the key identifying the image is transferred across the network during interactivity in most cases. The reduced network bandwidth increases the capability and interactivity of the application in lower bandwidth environments such as cellular data networks and other mobile platforms. In addition, through caching, the scalability of server systems may be greatly enhanced due to the reduced resource utilization (network bandwidth) and the decentralization of processing (image rendering and encoding).

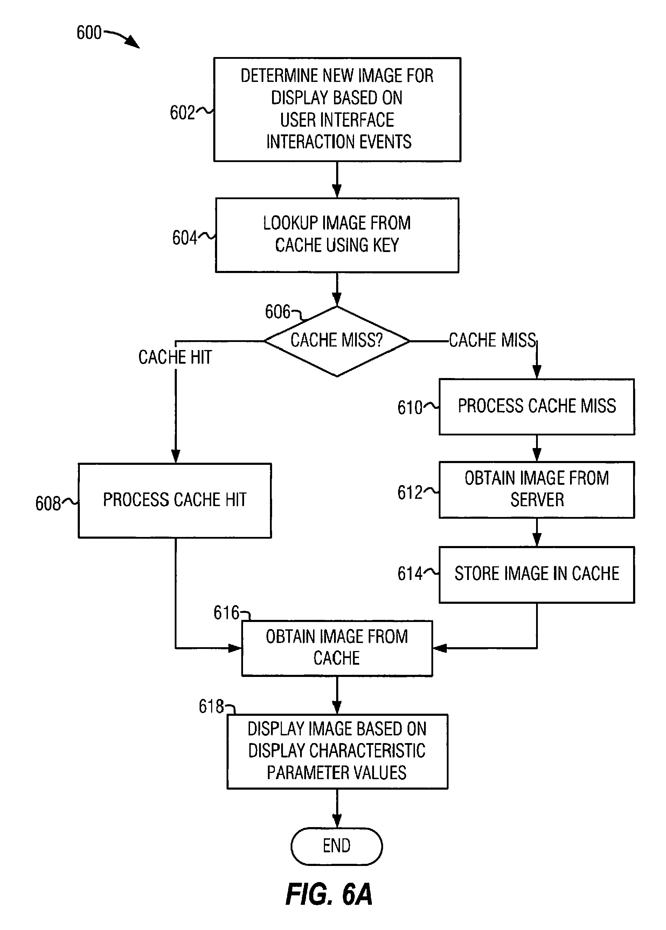

FIG. 6A illustrates a flowchart 600 for a technique of performing caching processing operations in a medical imaging viewer, from the perspective of client-side operations, according to the examples described herein. The operations in flowchart 600 include the request of an image for display based on a user interface interaction in the medical imaging viewer (operation 602). For example, user interaction in a graphical user interface may refer to the selection or navigation of a thumbnail, reduced size image, or pointer referring to a particular image in an image series. This new image may be requested from the server each time before a particular image is to be displayed on the client-side.

First, a lookup of the image is performed in the client-side cache, with use of a key that uniquely identifies the source image to be displayed (operation 604). A determination for a cache miss (decision 606) is performed, with different steps resulting from a client-side cache hit or cache miss. If a client-side cache hit occurs, the cache is processed (operation 608), and the image data is retrieved from a client-side cache (operation 616). If a client-side cache miss occurs, further operations are performed to obtain the data from the server. These further operations include processing of a cache miss (operation 610), obtaining or requesting image data from a server that matches the parameters of the cache miss (operation 612), loading (e.g., storing) the image in the cache (operation 614), and then obtaining the image from the client-side cache (operation 616). In other examples, the image is stored in the cache (operation 614) after its initial use and display by the image viewer.

Ultimately, the image is displayed based on display characteristic parameter values determined by the server (operation 618). Various image display operations may be performed at the client (e.g., in the browser) that change the parameters of the display image (such as zooming, panning, changing orientation, image, and the like). Subsequent operations to attempt a new display of the image may result in looking up the image from the cache and repeating the lookup and cache processing operations of flowchart 600.

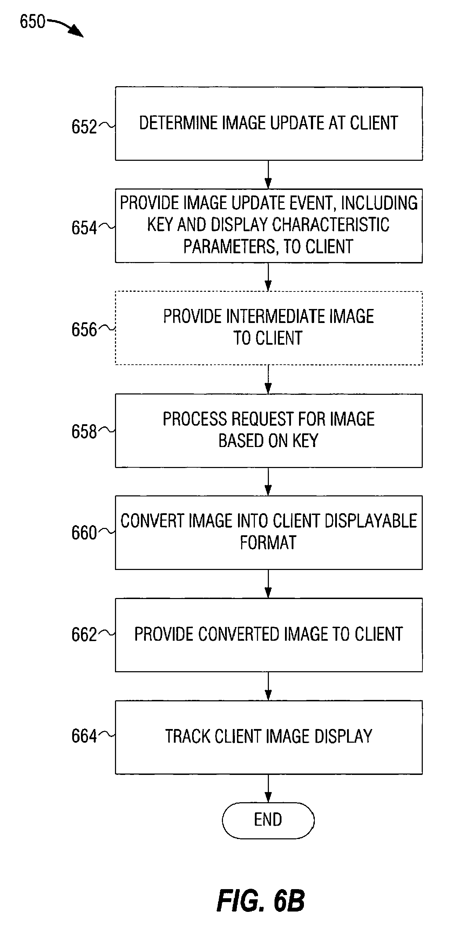

FIG. 6B illustrates a flowchart 650 of a technique for performing processing operations at a server supporting caching for a medical imaging viewer according to the examples described herein. The operations in flowchart 650 include various steps used to serve a particular image upon request by the client, such as when the client determines that the image is not cached locally. It will be understood that the following described operations for providing an intermediate image to the client (operation 656), converting the image (operation 660) and providing the converted image to the client (operation 662) might not occur in scenarios where the image is cached and retrieved locally at the client.

As shown in flowchart 650, the server processing of a request for a medical image occurs in response to the determination of an image update event at the client (operation 652), with the image update event occurring in response to processing of client user interface interaction events. From this, the server determines the characteristics of the image and the rendering parameters that inform the client of how to render the image. The server then provides the image update event to the client (operation 654), including providing a unique identifier correlated to the image (e.g., an image key) and display parameters (e.g., a display characteristic matrix) for the particular image display. In some examples, the image update event transmitted to the client may optionally include a pre-rendered intermediate (e.g., lower-resolution, lower-quality) image, inline to the initial response (operation 656). In other examples, the user interaction may be analyzed to determine whether the full converted image should be provided to the client to accompany the unique identifier and display parameters. For example, if the client browser supports in-line image data (data URIs), the full image data can be provided in the message to avoid an additional round trip (e.g., an additional response provided in operation 662).

The image unique identifier (e.g., key) is used to identify the image at the client, and support the client determination of whether the particular image is in the cache (e.g., operations in FIG. 6A). In a scenario where the image is not cached at the client, the server receives a subsequent request from the client for the image based on the identifier (operation 658). The full data for the image is converted (or has been previously converted) into a client-displayable format (operation 660). For example, this may include the conversion of a DICOM-format radiology image into a JPG/PNG browser-compatible format image. (In other examples not involving real-time conversion, the image key may be used to retrieve the image from a server-side cache.) The converted image is provided to the client (operation 662) in connection with the web server response, for example, as a data file transfer or a data URI response.

Further processing may involve modifications and tracking the client image display characteristics (operation 664), including tracking the characteristics provided in the display parameters provided in the image update event to the client. The operations for determining the image update at the client (operation 652) may also include evaluation of: a position of the viewable image in a display window, size characteristics of the viewable image in the display window, size and orientation characteristics of the viewable image in the display window, and the like. From these client image display characteristics, the server will perform various operations to maintain or otherwise track the characteristics of the client image display for the present or future user interface interactions.

In connection with the processing of the request for the medical image (operation 652), the server may be configured to generate images in real-time, on-the-fly, rather than with a previous conversion of all images for all series that may or may not be navigated. Thus, the server may be configured to generate the unique identifier (key) correlated to a particular image when the user interacts with the software to select a particular display of the medical image (and an image update is determined), as the server waits to perform a full conversion of the image until receiving a later client request for the image after the client cache is checked. Thus, in scenarios where the client already has the image in the cache or the client simply skips an image in an image series, unnecessary image conversion and accompanying CPU/memory/network operations can be avoided.

The previously described server-side processing techniques may also be implemented in connection with variations of prefetching/anticipatory strategies. For example, the server can generate the keys for a number of images located adjacent to or nearby the current image without expending much time, processing cycles, or memory. Pre-fetching can also be used to enable a client to load like adjacent or nearby images into its cache in the background. Such use of pre-fetching and caching on both the client and the server may significantly reduce the amounts of processing needed to access additional images. Other variations to the server-side and client-side caching and pre-fetching may also occur in connection with the use of the server/client synchronized unique identifier key and display parameters.

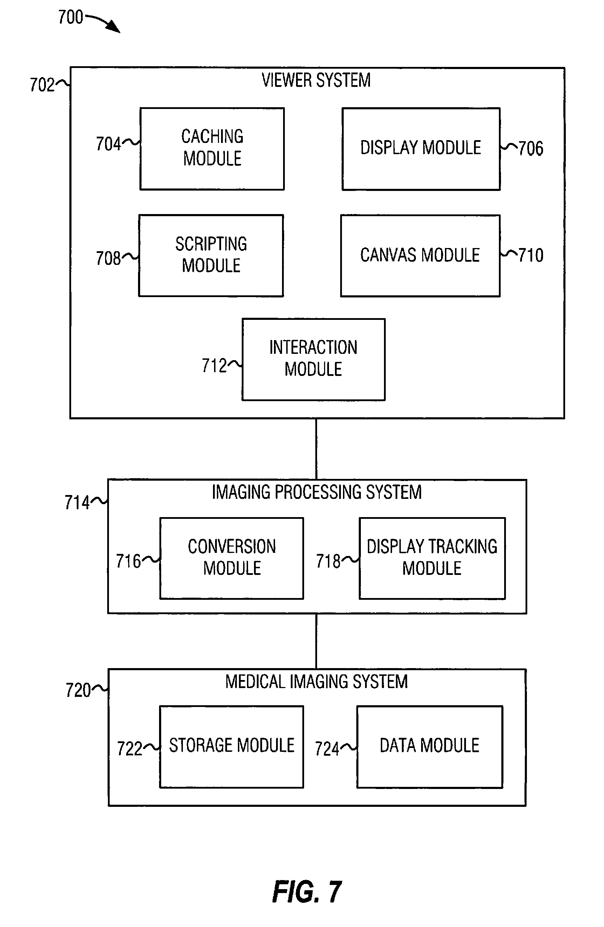

FIG. 7 illustrates an example configuration of a system architecture 700 configured to implement the presently described techniques for client-side image caching and server-side image processing. The system architecture 700 may include a viewer system 702, an image processing system 714, and a medical imaging system 720. Other systems and devices involved in the creation and processing of medical images are not depicted in FIG. 7, but are suitably integrated into operation with these components.

The viewer system 702 may include a caching module 704, display module 706, scripting module 708, canvas module 710, and interaction module 712. The caching module 704 may operate to implement a cache with the client-side caching techniques described above, similar to a browser-based cache, but maintaining copies of images indexed to an identifying key. The display module 706 may operate to provide a display of the image data in a graphical fashion, such as with the user of a graphical user interface in a web browser. The scripting module 708 may operate to process interaction requests in the display module and initiate contact to a remote server, in accordance with JavaScript and other client-side processing commands on the client. The canvas module 710 may operate to manage and present a display canvas of an image for output with the display module 706, for example, in a HTML5 canvas in a web browser. The interaction module 712 may operate in connection with the scripting module 708, display module 706, and canvas module 710, and include scripting or other functionality to detect when user interaction has requested a display update or other change to the displayed image.

The imaging processing system 714 may include a conversion module 716 and a display tracking module 718. The conversion module 716 may operate to perform conversion of image data and other data from an incompatible format (e.g., image modality output) into a display viewer compatible format (e.g., compatible for viewing by a browser at viewer system 702).

The medical imaging system 720 may include a storage module 722 and a data module 724. The storage module 722 may operate as an image archive, for example, providing information to the image processing system 714 on demand. The data module 724 of the medical imaging system 720 may operate as data processing for imaging information provided in other imaging systems. For example, the data module 724 may process information transmitted via the DICOM protocol to determine characteristics of medical imaging images in connection with the display and storage of the medical imaging data.

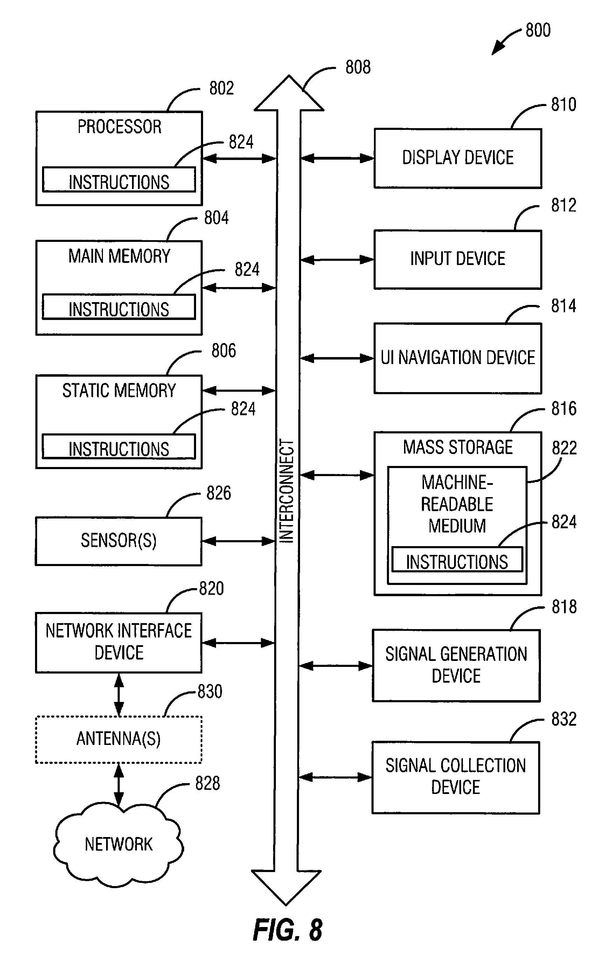

FIG 8 is a block diagram illustrating an example computing system machine upon which any one or more of the methodologies herein discussed may be run. Computer system 800 may be embodied as a computing device, providing operations of the components featured in the various figures, including components of the image processing server 110, client computer 120, browser 502, server 504, viewer system 702, imaging processing system 714, medical imaging system 716, or any other processing, storage, or computing platform or component described or referred to herein. In alternative embodiments, the machine operates as a standalone device or may be connected (e.g., networked) to other machines. In a networked deployment, the machine may operate in the capacity of either a server or a client machine in server-client network environments, or it may act as a peer machine in peer-to-peer (or distributed) network environments. The computer system machine may be a personal computer (PC) that may or may not be portable (e.g., a notebook or a netbook), a tablet, a Personal Digital Assistant (PDA), a mobile telephone or smartphone, a web appliance, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein.

Example computer system 800 includes a processor 802 (e.g., a central processing unit (CPU), a graphics processing unit (GPU) or both), a main memory 804 and a static memory 806, which communicate with each other via an interconnect 808 (e.g., a link, a bus, etc.). The computer system 800 may further include a video display unit 810, an alphanumeric input device 812 (e.g., a keyboard), and a user interface (UI) navigation device 814 (e.g., a mouse). In one embodiment, the video display unit 810, input device 812 and UI navigation device 814 are a touch screen display. The computer system 800 may additionally include a storage device 816 (e.g., a drive unit), a signal generation device 818 (e.g., a speaker), an output controller 832, and a network interface device 820 (which may include or operably communicate with one or more antennas 830, transceivers, or other wireless communications hardware), and one or more sensors 828.

The storage device 816 includes a machine-readable medium 822 on which is stored one or more sets of data structures and instructions 824 (e.g., software) embodying or utilized by any one or more of the methodologies or functions described herein. The instructions 824 may also reside, completely or at least partially, within the main memory 804, static memory 806, and/or within the processor 802 during execution thereof by the computer system 800, with the main memory 804, static memory 806, and the processor 802 also constituting machine-readable media.

While the machine-readable medium 822 is illustrated in an example embodiment to be a single medium, the term "machine-readable medium" may include a single medium or multiple media (e.g., a centralized or distributed database, and/or associated caches and servers) that store the one or more instructions 824. The term "machine-readable medium" shall also be taken to include any tangible medium that is capable of storing, encoding or carrying instructions for execution by the machine and that cause the machine to perform any one or more of the methodologies of the present disclosure or that is capable of storing, encoding or carrying data structures utilized by or associated with such instructions. The term "machine-readable medium" shall accordingly be taken to include, but not be limited to, solid-state memories, optical media, and magnetic media. Specific examples of machine-readable media include non-volatile memory, including, by way of example, semiconductor memory devices (e.g., Electrically Programmable Read-Only Memory (EPROM), Electrically Erasable Programmable Read-Only Memory (EEPROM)) and flash memory devices; magnetic disks such as internal hard disks and removable disks; magneto-optical disks; and CD-ROM and DVD-ROM disks.

The instructions 824 may further be transmitted or received over a communications network 826 using a transmission medium via the network interface device 820 utilizing any one of a number of well-known transfer protocols (e.g., HTTP). Examples of communication networks include a local area network (LAN), wide area network (WAN), the Internet, mobile telephone networks, Plain Old Telephone (POTS) networks, and wireless data networks (e.g., Wi-Fi, 3G, and 4G LTE/LTE-A or WiMAX networks). The term "transmission medium" shall be taken to include any intangible medium that is capable of storing, encoding, or carrying instructions for execution by the machine, and includes digital or analog communications signals or other intangible medium to facilitate communication of such software. A transmission medium is one embodiment of a machine readable medium.

Other applicable network configurations may be included within the scope of the presently described communication networks. Although examples were provided with reference to a local area wireless network configuration and a wide area Internet network connection, it will be understood that communications may also be facilitated using any number of personal area networks, LANs, and WANs, using any combination of wired or wireless transmission mediums.

The embodiments described above may be implemented in one or a combination of hardware, firmware, and software. While some embodiments described herein illustrate only a single machine or device, the terms "system", "machine", or "device" shall also be taken to include any collection of machines or devices that individually or jointly execute a set (or multiple sets) of instructions to perform any one or more of the methodologies discussed herein.

Examples, as described herein, may include, or may operate on, logic or a number of components, modules, or mechanisms. Modules are tangible entities (e.g., hardware) capable of performing specified operations and may be configured or arranged in a certain manner. In an example, circuits may be arranged (e.g., internally or with respect to external entities such as other circuits) in a specified manner as a module. In an example, the whole or part of one or more computer systems (e.g., a standalone, client or server computer system) or one or more hardware processors may be configured by firmware or software (e.g., instructions, an application portion, or an application) as a module that operates to perform specified operations. In an example, the software may reside on a machine readable medium. In an example, the software, when executed by the underlying hardware of the module, causes the hardware to perform the specified operations.

Accordingly, the term "module" is understood to encompass a tangible entity, be that an entity that is physically constructed, specifically configured (e.g., hardwired), or temporarily (e.g., transitorily) configured (e.g., programmed) to operate in a specified manner or to perform part or all of any operation described herein. Considering examples in which modules are temporarily configured, each of the modules need not be instantiated at any one moment in time. For example, where the modules comprise a general-purpose hardware processor configured using software, the general-purpose hardware processor may be configured as respective different modules at different times. Software may accordingly configure a hardware processor, for example, to constitute a particular module at one instance of time and to constitute a different module at a different instance of time.

Additional examples of the presently described method, system, and device embodiments are suggested according to the structures and techniques described herein, and specified in the following claims. For example, the subject matter described herein may be embodied by a method performed by a client device (e.g., a computer system) for client-side caching of medical images, performed by a computing device having at least one processor and at least one memory, and the method implemented by operations performed using the processor and the memory.

As another example, the subject matter described herein may be embodied by a server-performed method for supporting client-side caching of medical images, performed by a computing device having at least one processor and at least one memory, the method implemented by operations performed using the processor and the memory.

As yet another example, the subject matter described herein may be embodied by a machine-readable medium, the machine-readable medium including instructions, which when executed by a machine having a hardware processor, causes the machine to perform operations of the client- or server-performed method(s).

As yet another example, the subject matter described herein may be embodied by a an imaging processing system, comprising: at least one processor and at least one memory; a conversion module configured to perform server-side conversion of medical images requested by a client viewer system; and a display tracking module configured to provide image display parameters for the client viewer system according to caching operations occurring at the client viewer system, the conversion module and the display tracking module further configured to implement operations of the server-performed method.

As yet another example, the subject matter described herein may be embodied by a medical imaging viewer system, comprising: at least one processor and at least one memory; a display module configured to output a medical image display interface; and a caching module configured to perform a lookup of the image data for the medical image in a cache.

Other non-limiting examples may be configured to operate separately, or can be combined in any permutation or combination with any one or more of the other examples provided above, in the following claims, or throughout the present disclosure.

## Claims

1. A method for client-side caching of a medical image, performed by a client computing device having at least one processor and at least one memory, the method implemented by operations performed using the at least one processor and the at least one memory, with the operations comprising:
processing user interaction received in a medical image display interface on the client computing device, the user interaction selecting a display of a medical image within the medical image display interface;
receiving, from a server in response to selecting the display of the medical image, a unique identifier correlated to image data of the medical image; and
performing a lookup of image data for the medical image in a cache accessible to the client computing device, the lookup of the image data for the medical image being performed using the unique identifier of the medical image.

2. The method of claim 1, the operations comprising:
retrieving the image data for the medical image from the cache, wherein the retrieval of the image data is performed with use of the unique identifier; and
displaying at least a portion of the medical image using the image data retrieved from the cache.

3. The method of claim 1 or claim 2, wherein the user interaction is used to result in a change to a medical image display at the client computing device, and wherein the image data is used to effect the change to the medical image display.

4. The method of any preceding claim, the operations comprising:
receiving, from the server in response to selecting the display of the medical image, a plurality of display parameters of the medical image for the display; and
displaying the image data for the medical image in the medical image display interface, the displaying of the image data implemented using the plurality of display parameters received from the server.

5. The method of claim 4, wherein the medical image display interface is provided within a browser executing on the client computing device using the processor and the memory, the browser in operable communication with the server via a network connection with the client computing device.

6. The method of claim 4 or claim 5, wherein the plurality of display parameters include parameters for a specific display output of the medical image in the medical image display interface, the parameters specifying image position, image size, image orientation, and image rotation.

7. The method of claim 6, wherein the user interaction selecting the display of the medical image indicates a change to the display of at least one particular medical image in a medical image series, the change to the display incorporating a change to: the image position, the image size, the image orientation, or the image rotation, of the at least one particular medical image.

8. The method of any preceding claim, the operations comprising:
processing a cache miss from the lookup of the image data in the cache;
obtaining the image data from the server using the unique identifier; and
storing the image data in the cache.

9. The method of any preceding claim, the operations comprising:
processing a cache hit from the lookup of the image data in the cache; and
obtaining the image data from the cache using the unique identifier.

10. The method of any preceding claim, the operations comprising:
receiving, from the server in response to selecting the display of the medical image, a plurality of display parameters of the medical image for the display;
receiving, from the server in response to selecting the display of the medical image, image data for an intermediate medical image, the intermediate medical image being provided inline in a server response, wherein the intermediate medical image is a representation of the medical image, and wherein the unique identifier and a plurality of display parameters accompany the intermediate medical image in the server response;
displaying image data for the intermediate medical image in the medical image display interface according to at least one of the plurality of display parameters received from the server;
obtaining the image data for the medical image, in response to the performing the lookup of the medical image in the cache; and
displaying the image data for the medical image in the medical image display interface according to the plurality of display parameters received from the server, wherein the display of the image data for medical image replaces the display of the image data for the intermediate medical image.

11. The method of any preceding claim, the operations comprising:
transmitting, to the server in response to selecting the display of the medical image via a user interaction, user interface interaction information, the user interaction occurring on a first medical image in the medical image display interface; and
receiving, from the server in response to the user interface interaction information, a new image event indicating selecting a display of a second medical image, wherein the image data is used to fulfill a display of the second medical image;
wherein the first medical image and the second medical image are images within a plurality of images in a medical imaging series, the medical imaging series including a plurality of images from a radiology imaging procedure.

12. A machine readable medium including code, that when executed, causes a machine to perform the method of any preceding claim.

13. An apparatus comprising means for performing the method of any one of claims 1-11.

14. A medical imaging viewer device, comprising:
a display screen adapted to output a medical image display interface;
a memory adapted to maintain a cache of medical image data for the medical image display interface;
at least one processor executing instructions to control the medical image display interface, the instructions causing the medical image display interface to:
receive user interaction, the user interaction specifying a change to a display output within the medical image display interface; and
display image data for the medical image, the display of the image data occurring according to a plurality of display parameters received from a server;
perform a lookup of the image data for the medical image in the cache, the lookup of the image data for the medical image being performed based on a unique identifier of the medical image received from the server;
wherein the plurality of display parameters received from the server include parameters for display in the medical image display interface, the parameters including image position, image size, image orientation, and image rotation, and wherein the change to the display output within the medical image display interface includes a change of a display of a single medical image in a medical image series, the change of the display incorporating a change to: the image position, the image size, the image orientation, or the image rotation, of the single medical image.

15. The system of claim 14, further comprising instructions that cause the medical image display interface to process a cache hit and a cache miss for the image data, the cache miss resulting in a communication to an image processing server for retrieval of the image data, and the cache hit resulting in a retrieval of the image data from the cache, wherein the cache miss further results in storage of the image data in the cache in response to the retrieval of the image data from the image processing server.
